# EUROPEAN PATENT APPLICATION

(11) **EP 3 153 974 A1**
(43) Date of publication of application: **12.04.2017**
(21) Application number: 14894120.6
(22) Date of filing: 04.06.2014
(51) Int. Cl.: G06F 17/30, G06Q 50/24

(54) **MEDICAL CARE DATA SEARCH SYSTEM**

(71) Applicant: Hitachi, Ltd., Chiyoda-ku Tokyo 100-8280 (JP)
(72) Inventor: KIDO Kunihiko, Tokyo 100-8280 (JP)
(74) Representative: Addiss, John William
(86) International application number: PCT/JP2014/064822
(87) International publication number: WO 2015/186205

(57) **Abstract**

A medical care data retrieval system includes a storage device including a medical care data database in which medical care contents for symptoms of patients are stored and a medical care pattern database in which relations between the symptoms and the medical care contents are stored and a graph processing unit that creates, on the basis of the medical care data database and the medical care pattern database, a graph including a root node representing the symptom, at least one intermediate node representing a name of the medical care content, and at least one leaf node representing a reference number of the medical care content. When there are a plurality of the graphs concerning a certain symptom, the graph processing unit integrates redundant portions of the intermediate node among the plurality of graphs.

## Description

### Technical Field

The present invention relates to a retrieval system for retrieving medical care data.

### Background Art

As a background art of this technical field, there is Patent Literature 1. Patent Literature 1 discloses a retrieval system for a chemical compound having a tree structure. In this retrieval system, a tree structure formed by nodes corresponding to atoms and edges corresponding to interatomic bonds is represented and a partial structure of a chemical compound is converted into a character string on the basis of the tree structure to facilitate retrieval.

### Citation List

### Patent Literature

Patent Literature 1: JP-A-2007-323182

### Summary of Invention

### Technical Problem

Since medical care data is high dimensional and relationship among data is complicated, it is necessary to divide data items into a plurality of tables and manage the data items. Therefore, when a data set serving as an analysis target is prepared, it is necessary to combine the plurality of tables. In general, the table combination has a large load. When a large amount of data is treated, a processing time for the data is a problem. In particular, medical care data has a large data amount and has a complicated table structure. Therefore, table combination in the medical care data has a large load. Efficiency of retrieval is a problem.

An object of the invention is to provide a retrieval system that makes it possible to perform table combination at high speed and achieve improvement of efficiency of retrieval in medical care data having a complicated table structure.

### Solution to Problem

In order to achieve the object, for example, a configuration described in claims is adopted. This application includes a plurality of means for solving the problems. As an example of the means, there is provided a medical care data retrieval system including: a storage device including a medical care data database in which medical care contents for symptoms of patients are stored and a medical care pattern database in which relations between the symptoms and the medical care contents are stored; and a graph processing unit that creates, on the basis of the medical care data database and the medical care pattern database, a graph including a root node representing the symptom, at least one intermediate node representing a name of the medical care content, and at least one leaf node representing a reference number of the medical care content. When there are a plurality of the graphs concerning a certain symptom, the graph processing unit integrates redundant portions of the intermediate node among the plurality of graphs.

### Advantageous Effect of Invention

According to the invention, even in medical care data having a complicated table structure, it is possible to perform table combination at high speed and it is possible to achieve improvement of efficiency of retrieval.

Further characteristics related to the invention will become clear from the description of this specification and the accompanying drawings. Problems, configurations, and effects other than those explained above are made clear from the following explanation of embodiments.

### Brief Description of the Drawings

[Fig. 1] Fig. 1 is a configuration diagram of a medical care data retrieval system of a first embodiment.
[Fig. 2] Fig. 2 is a flowchart of the system of the first embodiment.
[Fig. 3] Fig. 3 is an example of a target episode table.
[Fig. 4] Fig. 4 is an example of a patient table.
[Fig. 5] Fig. 5 is an example of an episode table.
[Fig. 6] Fig. 6 is an example of a medical practice table.
[Fig. 7] Fig. 7 is an example of an examination result table.
[Fig. 8] Fig. 8 is an example of a prescription table.
[Fig. 9] Fig. 9 is an example of a related table.
[Fig. 10] Fig. 10 is an example of a data structure concerning diagnostic treatment knowledge.
[Fig. 11] Fig. 11 is an example of an examination-adapted disease table.
[Fig. 12] Fig. 12 is an example of a drug-adapted disease table.
[Fig. 13] Fig. 13 is an example of a medical care pattern serial number management table.
[Fig. 14] Fig. 14 is an example of an examination serial number management table.
[Fig. 15] Fig. 15 is an example of a drug serial number management table.
[Fig. 16] Fig. 16 is an example of a medical practice serial number management table.
[Fig. 17] Fig. 17 is an example of a connected graph.
[Fig. 18] Fig. 18 is another example of the connected graph.
[Fig. 19A] Fig. 19A is a first example of a reference tree by the connected graph.
[Fig. 19B] Fig. 19B is a second example of the reference tree by the connected graph.
[Fig. 20] Fig. 20 is an example in which the two connected graphs of Fig. 19A and Fig. 19B are integrated.
[Fig. 21A] Fig. 21A is an example in which node names in Fig. 20 are replaced with serial numbers.
[Fig. 21B] Fig. 21B is an example in which leaf nodes in Fig. 21A are sorted.
[Fig. 22] Fig. 22 is an example in which differences are calculated for the serial numbers of the nodes in Fig. 21B.
[Fig. 23] Fig. 23 is an example in which the nodes in Fig. 22 are encoded.
[Fig. 24] Fig. 24 is a flowchart for explaining related table generation in the first embodiment.
[Fig. 25] Fig. 25 is a flowchart for explaining retrieval processing in the first embodiment.
[Fig. 26] Fig. 26 is a hardware system configuration example related to the first embodiment.
[Fig. 27] Fig. 27 is a flowchart for explaining related table generation in a second embodiment.
[Fig. 28] Fig. 28 is a flowchart for explaining retrieval processing in the second embodiment.
[Fig. 29] Fig. 29 is an output example of a retrieval result.

### Description of Embodiments

Embodiments of the invention are explained below with reference to the accompanying drawings. Note that the accompanying drawings show specific embodiments conforming to the principle of the invention. However, the accompanying drawings are for understanding of the invention and are not used to limitedly interpret the invention.

### [First Embodiment]

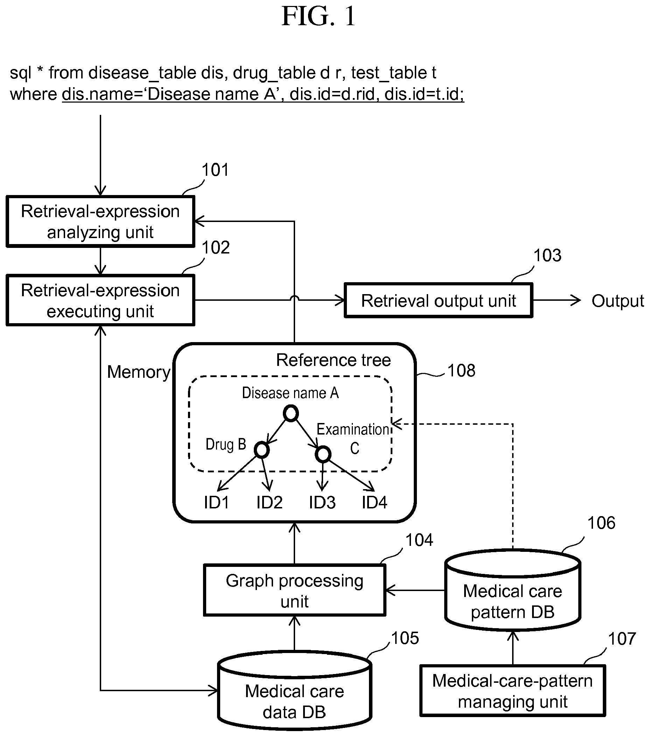

Fig. 1 shows the configuration of an entire medical care data retrieval system of this embodiment. The medical care data retrieval system includes a retrieval-expression analyzing unit 101, a retrieval-expression executing unit 102, a retrieval output unit 103, a graph processing unit 104, a medical care data database (DB) 105, a medical care pattern database (DB) 106, and a medical-care-pattern managing unit 107.

The retrieval-expression analyzing unit 101 analyzes a retrieval sentence (e.g., a SQL sentence described in Fig. 1) input by a user and passes a retrieval execution schedule to the retrieval-expression executing unit 102. The retrieval-expression executing unit 102 retrieves data from the medical care data DB 105 on the basis of the retrieval execution schedule and passes a examination result to the retrieval output unit 103. The retrieval output unit 103 outputs the retrieval result.

In drawing up the retrieval execution plan, the retrieval-expression analyzing unit 101 refers to a graph (a reference tree) 108 present on a memory and looks for a position of a record matching retrieval conditions of the retrieval sentence. The graph 108 is generated by the graph processing unit 104 on the basis of the medical care data DB 105 and the medical care pattern DB 106.

The medical care DB 105 is a database in which medical care contents for episodes of patients are recorded. The episode means development of a certain symptom. The medical care contents include a medical practice for a patient, a drug administered to the patient, and contents of an examination for the patient. Information of the medical care data DB 105 is provided from a data provider such as a medical institution. The medical care pattern DB 106 is a database that defines relations between symptoms and medical care contents. The medical care pattern DB 106 stores knowledge known in the medical field.

The medical-care-pattern managing unit 107 is a processing unit for storing input various data in the medical care pattern DB 106. A data administrator can appropriately edit and manage the information of the medical care pattern DB 106 via the medical-care-pattern managing unit 107.

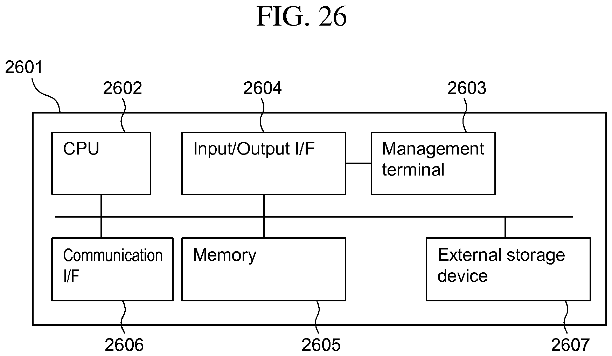

A hardware system configuration for realizing Fig. 1 is explained. Fig. 26 is a hardware system configuration diagram concerning the medical care data retrieval system according to the first embodiment.

The medical care data retrieval system is realized by executing a program in a computer 2601 such as a data center or a server. For example, the retrieval-expression analyzing unit 101, the retrieval-expression executing unit 102, the retrieval output unit 103, the graph processing unit 104, and the medical-care-pattern managing unit 107 can be realized by a processing interpreting and executing programs for realizing functions of the units.

The computer 2601 is configured from a CPU 2602, which is an arithmetic unit that executes programs and processes data, a management terminal 2603, an input/output interface 2604 that configures an interface with the management terminal 2603, a memory 2605 that stores the programs, the data, and the like, a communication interface 2606 that configures an interface with the outside, an external storage device 2607 (the DBs 105 and 106 in Fig. 1 are disposed here), and the like.

The management terminal 2603 includes an input unit such as a mouse or a keyboard and an output unit such as a display. An operator can input various kinds of input information via the input unit. The input information, a retrieval result, and the like are displayed on the output unit.

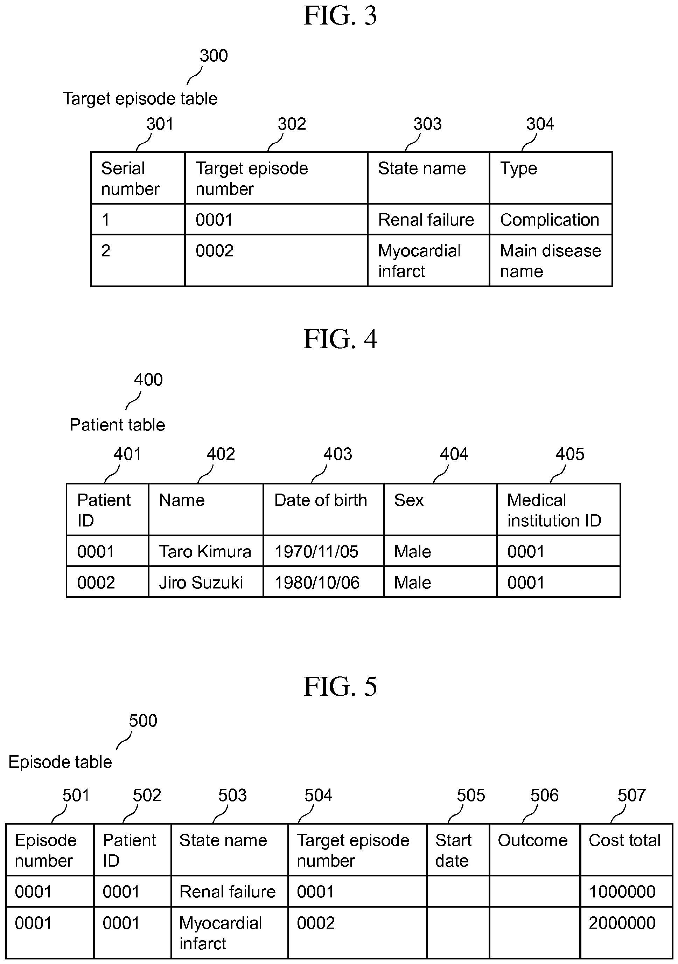

A data configuration treated by the system is explained below. Fig. 3 shows a target episode table. A target episode table 300 is a master table for managing episodes serving as processing targets of the graph processing unit 104. The target episode table 300 includes, as constituent items, a serial number 301, which is a serial number of an individual entry, a target episode number 302 for uniquely specifying an episode, a state name 303 representing the episode, and a type 304 indicating whether the episode is a main disease name or a complication. The state name 303 includes a disease name, a complication, or a comorbidity.

For example, the target episode table 300 is created by the data administrator operating the management terminal 2603. The target episode table 300 may be stored in the medical care data DB 105 via the retrieval-expression analyzing unit 101 and the retrieval-expression executing unit 102 using a retrieval sentence (SQL) for data writing. The data administrator creates the target episode table 300 and designates a target of data to be collected.

Fig. 4 shows a patient table. A patient table 400 is a master table for managing basic information of patients. The patient table 400 includes, as constituent items, a patient ID 401 for identifying a patient, a name 402 of the patient, a date of birth 403 of the patient, sex 404 of the patient, and a medical institution ID 405, which is an identifier for identifying a medical institution where the patent receives a medical care.

Fig. 5 shows an episode table. An episode table 500 is a table in which information concerning episodes (symptoms) that actually occur in patients is stored. The episode table 500 includes, as constituent items, an episode number 501, which is a number for specifying an episode, a patient ID 502 for identifying a patient, a state name 503 representing the episode, a target episode number 504 for referring to a record corresponding to the state name from the target episode table 300 in Fig. 3, a start date 505 of the episode, an outcome 506 indicating a consequence of the episode such as a cure or death, and a cost total 507, which is a total value of costs related to the episode. Note that the target episode number 504 is a null value when a corresponding state name is absent in the target episode table 300 in Fig. 3.

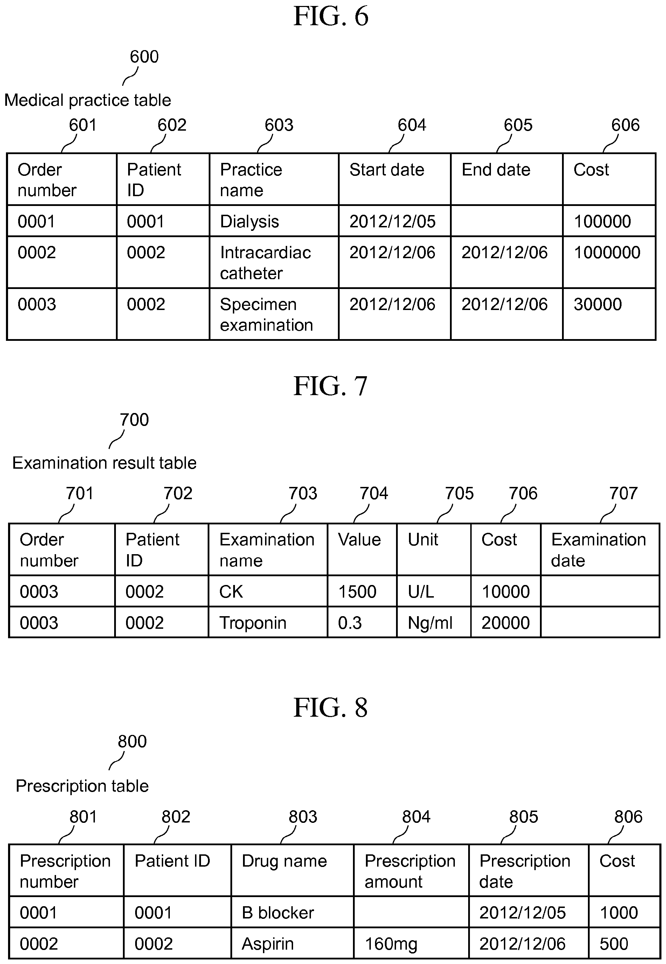

Fig. 6 shows a medical practice table. A medical practice table 600 is a table in which information concerning medical practices actually applied to patients is stored. The medical practice table 600 includes, as constituent items, an order number 601, which is a serial number of an individual entry, a patient ID 602 for identifying a patient, a practice name 603 concerning a medical practice carried out on the patient, a start date 604 and an end date 605 concerning the medical practice, and cost 606 concerning the medical practice. In the practice name 603, names concerning medical practices such as an examination, a treatment, and a surgical operation are described.

Fig. 7 shows an examination result table. An examination result table 700 is a table for managing results concerning specimen examinations entered in the medical practice table 600 in Fig. 6. The examination result table 700 includes, as constituent items, an order number 701 for specifying which specimen examination result in the medical practice table 600 an examination result is, a patient ID 702 for identifying a patient, an examination name 703, which is a name of an examination, a value 704, which is an examination result numerical value of the examination name, a unit 705 of the value, cost 706 concerning the examination, and an examination date 707.

A plurality of examinations are sometimes carried out concerning one specimen examination. Therefore, a plurality of records are sometimes present concerning the same order number. Fig. 7 indicates a case in which two examinations are included in one order number.

Fig. 8 shows a prescription table. A prescription table 800 is a table in which information concerning drugs prescribed for patients is stored. The prescription table 800 includes, as constituent items, a prescription number 801, which is a serial number of an individual entry, a patent ID 802 for identifying a patient, a drug name 803 concerning a prescribed drug, a prescription amount 804 of the drug, a prescription date 805, and cost 806 concerning the prescription.

For example, the data of Fig. 4 to Fig. 8 can be obtained from a data provider such as a medical institution. The data is stored in the medical care data DB 105 via the retrieval-expression analyzing unit 101 and the retrieval-expression executing unit 102 using a retrieval sentence (SQL) for data writing. The medical care data DB 105 is managed in the external storage device 2607.

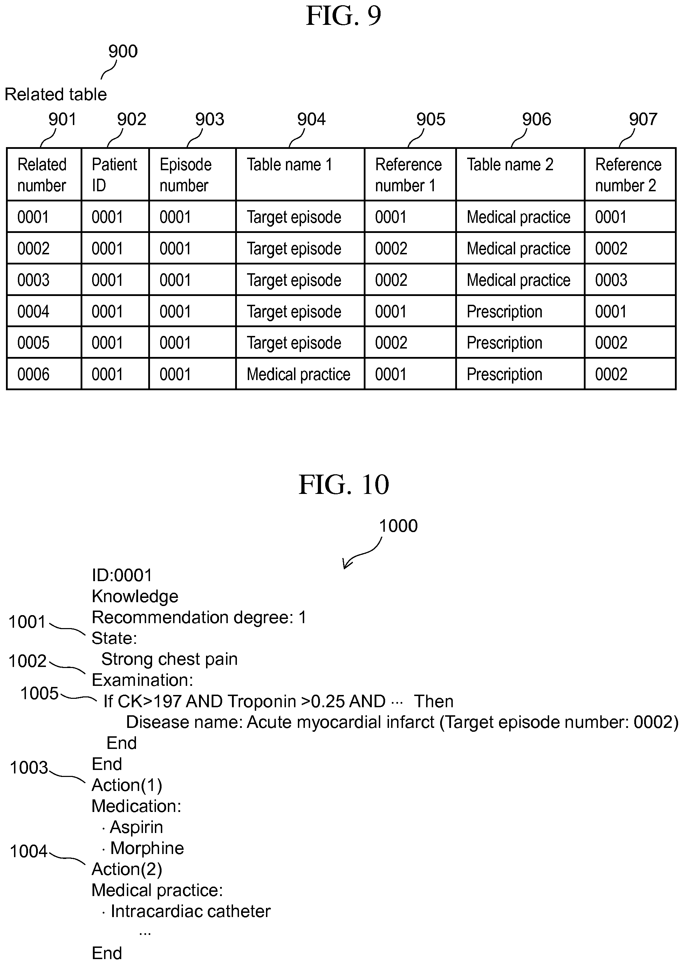

Fig. 6, Fig. 7, and Fig. 8 are records concerning medical care contents (a medical practice, an examination, prescription of a drug) performed on a certain patient. However, only with these tables, it cannot be determined to which episodes medical care contents relate. Fig. 9 shows a related table used for specifying relations between episodes of patents and medical care contents.

A related table 900 includes information indicating relations between episodes of patients and medical care contents and information indicating relations among the medical care contents carried out on the patients. The related table 900 includes, as constituent items, a related number 901, which is a serial number of an individual entry, a patient ID 902 for identifying a patient, an episode number 903 for referring to an episode serving as a target from the episode table in Fig. 5, a table name 1 (904) for designating a table, a reference number 1 (905) indicating a reference number in the table name 1 (904), a table name 2 (906) for designating a table, and a reference number 2 (907) indicating a reference number in the table name 2 (906).

In this example, when 904 and 906 are a "target episode", the reference numbers are the target episode number 504 in Fig. 5. When 904 and 906 are a "medical practice", the reference numbers are the order number 601 in Fig. 5. When 904 and 906 are an "examination result", the reference numbers are the order number 701 in Fig. 7. When 904 and 906 are "prescription", the reference numbers are the prescription number 801 in Fig. 8.

As shown in Fig. 9, in the related table 900, medical care content is associated with an episode in some case and medical care contents are associated with each other in other cases. When the medical care contents are associated with each other, as an example, medical care content indicated by the table name 1 (904) and the reference number 1 (905) may be defined as being carried out temporally before medical care content indicated by the table name 2 (906) and the reference number 2 (907).

Fig. 10 shows an example of diagnostic treatment knowledge. Diagnostic treatment knowledge 1000 has the structure of a file. The diagnostic treatment knowledge 1000 is a file having a predetermined structure such as an XML. In the diagnostic treatment knowledge 1000 of this example, an examination carried out when a patient is in a certain state, a disease name determined from a result of the examination, a medical practice carried out in that state, and the like are defined.

The diagnostic treatment knowledge 1000 includes, as constituent items, a state section 1001 representing a symptom ("strong chest pain", etc.) of a patient, an examination section 1002 representing an examination normally performed in the case of the symptom and a disease name determined from a result of the examination, a medical care content section 1003 representing first medical care content performed on the patient in the state, and a medical care content section 1004 representing second medical care content performed on the patient in the state.

In the examination section 1002, conditions 1005 (CK>197, troponin>0.25, AND ...) for determining a disease name from an examination result are defined. Therefore, when the conditions 1005 for the test result are satisfied, a disease name is specified. In this example, a portion of the disease name is associated with the target episode number 302 of the target episode table 300 in Fig. 3.

In the medical care content section 1003, drug names such as "aspirin" and "morphine" administered to the patient are defined. In the medical care content section 1004, medical practice names such as "intracardiac catheter" normally performed in the state are defined. Note that (1) and (2) of Action (1) of the medical care content section 1003 and Action (2) of the medical care content section 1004 indicate time order. In this example, it is indicated that the medical care content section 1003 has to be carried out before the medical care content section 1004.

These kinds of diagnostic treatment knowledge are knowledge that is a widely-recognized established theory in the medical field. The data administrator stores such knowledge for various symptoms in the medical care pattern DB 106 in advance via the medical-care-pattern managing unit 107 using, for example, the management terminal 2603. The diagnostic treatment knowledge is used to create a reference tree. Note that the medical care pattern DB 106 is managed in the external storage device 2607.

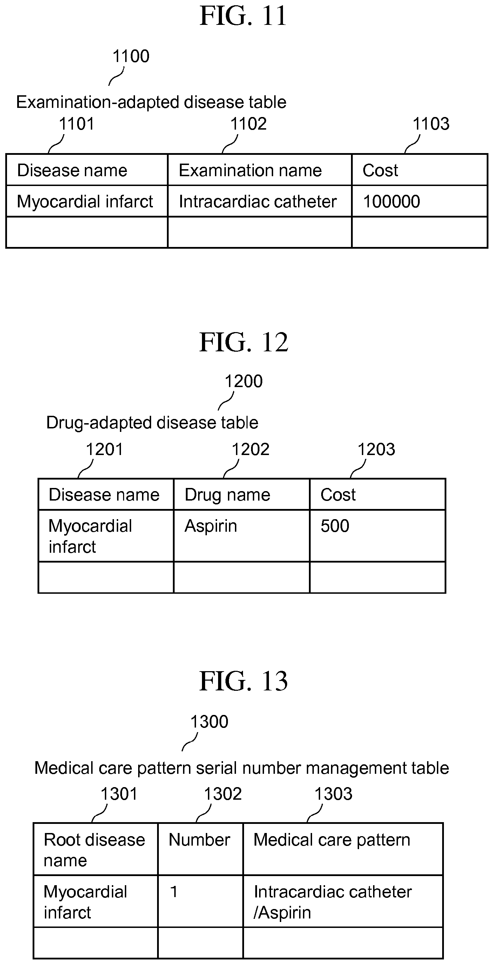

Fig. 11 shows an examination-adapted disease table. An examination-adapted disease table 1100 is a master table for managing adapted diseases of examinations and is one kind of the diagnostic treatment knowledge. The examination-adapted disease table 1100 includes, as constituent items, a disease name 1101, an examination name 1102 adapted to the disease name, and cost 1103 concerning the examination. A certain examination being adapted to a disease name means that, if the disease name (1101) is suspected, there is validity in implementation of the examination.

Fig. 12 shows a drug-adapted disease table. A drug-adapted disease table 1200 is a master table for managing adapted diseases of drugs and is a kind of the diagnostic treatment knowledge. The drug-adapted disease table 1200 includes, as constituent items, a disease name 1201, a drug name 1202 adapted to the disease name, and cost 1203 concerning the drug. A certain drug being adapted to a certain disease name means that, if the disease name is suspected, there is validity in prescription of the drug.

Note that, as in Fig. 10, the examination-adapted disease table 1100 and the drug-adapted disease table 1200 are stored in the medical care pattern DB 106 in advance via the medical-treatment-pattern managing unit 107. The diagnostic treatment knowledge is used to create a reference tree.

Fig. 13 shows a medical care pattern serial number management table. A medical care pattern means a combination of medical care contents (a medical practice, a drug, an examination, etc.). A medical care pattern serial number management table 1300 is a table for managing serial numbers of medical care patterns. As explained in detail below, in this embodiment, character strings of node names of a reference tree are replaced with numbers in order to compress a data amount.

The medical care pattern serial number management table 1300 defines relations between names at the time when nodes are medical care patterns and numbers for replacing the names. The medical care pattern serial number management table 1300 includes, as constituent items, a root disease name 1301, a number 1302 representing a serial number, and a medical care pattern 1303 representing a medical care pattern name. In an example shown in Fig. 13, information concerning a combination of "intracardiac catheter" and "aspirin" is stored.

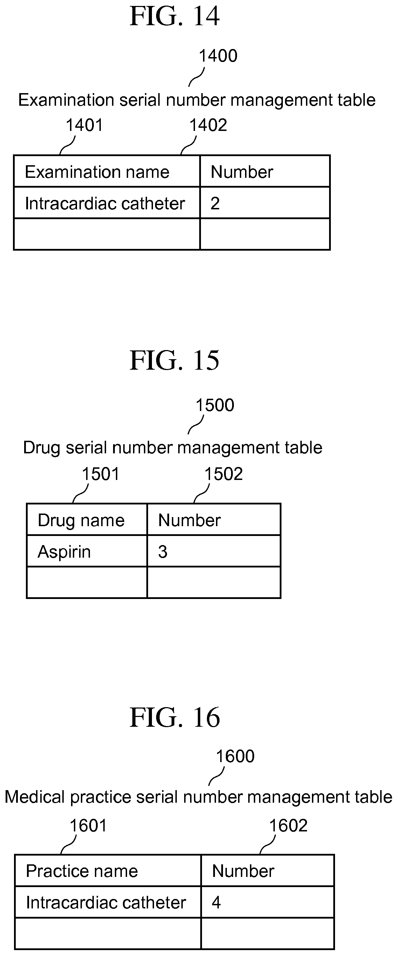

Fig. 14 shows an examination serial number management table. An examination serial number management table 1400 is a table for managing serial numbers of examination names. The examination serial number management table 1400 includes, as constituent items, an examination name 1401 and a number 1402 representing a serial number.

Fig. 15 shows a drug serial number management table. A drug serial number management table 1500 is a table for managing serial numbers of drug names. The drug serial number management table 1500 includes, as constituent items, a drug name 1501 and a number 1502 representing a serial number.

Fig. 16 shows a medical practice serial number management table. A medical practice serial number management table 1600 is a table for managing serial numbers of medical practice names. The medical practice serial number management table 1600 includes, as constituent items, a practice name 1601 and a number 1602 representing a serial number.

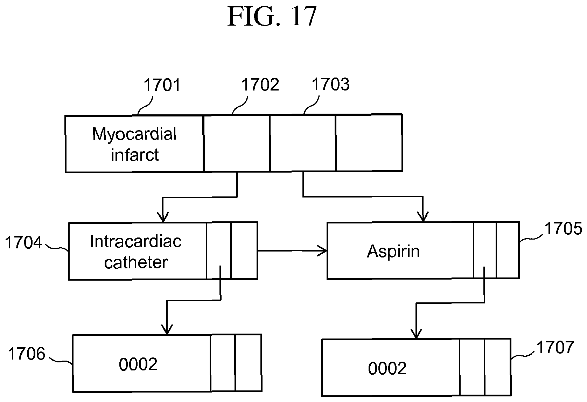

Fig. 17 shows the structure of a connected graph for storing a reference tree. The graph includes a root node representing a symptom, at least one intermediate node representing a name of medical care content, and at least one leaf node representing a reference number of the medical care content.

The structure of nodes is explained. Reference numeral 1701 denotes an area for storing a value of a node and 1702 and 1703 are areas for storing pointers of the next nodes. In the figure, "myocardial infarct" is set as a root node. The root node is linked to an intermediate node 1704 of "intracardiac catheter" by the pointer 1702. Similarly, the root node is linked to an intermediate node 1705 of "aspirin" by the pointer 1703.

Leaf nodes 1706 and 1707 respectively store reference numbers of records of tables related to the intermediate nodes 1704 and 1705 higher in order than the leaf nodes 1706 and 1707. For example, information concerning the order number 601 of the medical practice table 600 is stored in the leaf node 1706. Information concerning the prescription number 801 of the prescription table 800 is stored in the leaf node 1707.

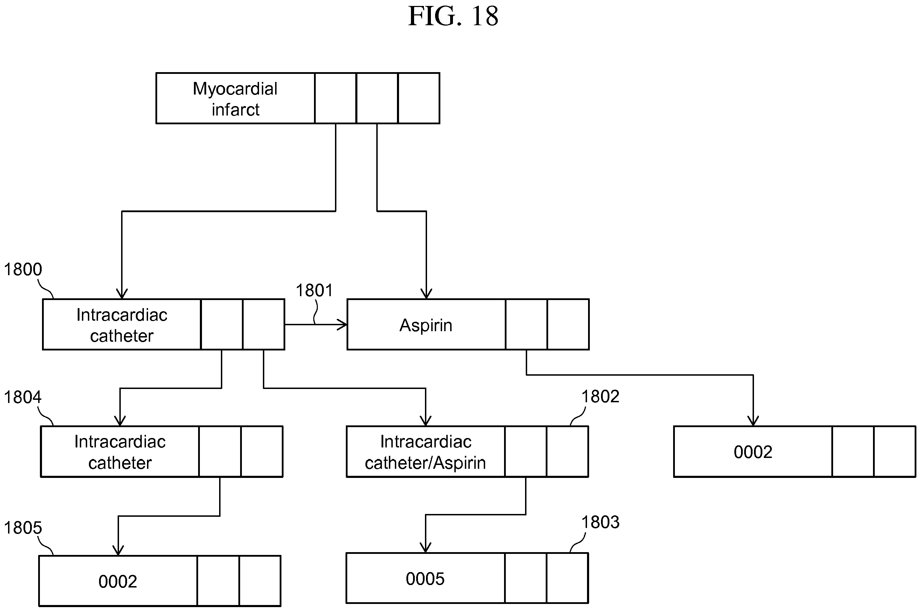

Like Fig. 17, Fig. 18 shows the structure of a connected graph for storing a reference tree. In Fig. 18, a link 1801 is present from a medical practice node to a prescription node. A case is explained below in which, as indicated by the link 1801, there is a medical relation between an "intracardiac catheter" node and an "aspirin" node.

There are two cases concerning a medical practice of the medical practice node; a case in which prescription of the prescription node is carried out after the medical practice and a case in which the prescription is not carried out. In the graph structure shown in Fig. 17, this relation cannot be distinguished. In other words, an AND condition in the case in which an arrow is present between the nodes, in this case, a state of "the medical practice and the prescription" cannot be represented by the graph structure of Fig. 17.

Therefore, in this embodiment, when a plurality of intermediate nodes are associated by a link representing temporal anteriority and posteriority, second intermediate nodes representing a pattern of a combination of medical care contents of the plurality of intermediate nodes are created under the intermediate nodes. Leaf nodes representing reference numbers of the pattern of the combination of the medical care contents are created under the second intermediate nodes.

As shown in Fig. 18, second intermediate nodes (relay nodes) 1802 and 1804 are provided and leaf nodes 1803 and 1805, which store reference numbers, are suspended under the second intermediate nodes 1802 and 1804. The host node 1800 retains pointers to the second intermediate nods 1802 and 1804. A name of the second intermediate node 1802 is "intracardiac catheter/aspirin", which indicates that "aspirin" is prescribed after a medical practice of "intracardiac catheter".

A reference number of the leaf node 1803 of "intracardiac catheter/aspirin" is "0005". For example, in the medical practice table 600, a record of the "intracardiac catheter" in the case in which the aspirin is not prescribed thereafter and a record of the "intracardiac catheter" in the case in which the aspirin is prescribed thereafter could be present. In the leaf node 1803, a reference number of the record of the "intracardiac catheter" in the case in which the aspirin is prescribed is stored.

On the other hand, a name of the second intermediate node 1804 is "intracardiac catheter", which indicates that there is no prescription related to the link 1801 after the medical practice of the "intracardiac catheter". A reference number of the leaf node 1805 in this case is "0002", which is a reference number of the "intracardiac catheter" in the case in which the aspirin is not prescribed. By providing such second intermediate nodes 1802 and 1804, it is possible to realize, as graph representation, the AND condition in the case in which the link is present between the nodes.

The serial number management tables shown in Fig. 13 to Fig. 16 and the connected graphs shown in Fig. 17 and Fig. 18 are managed on the memory 2605.

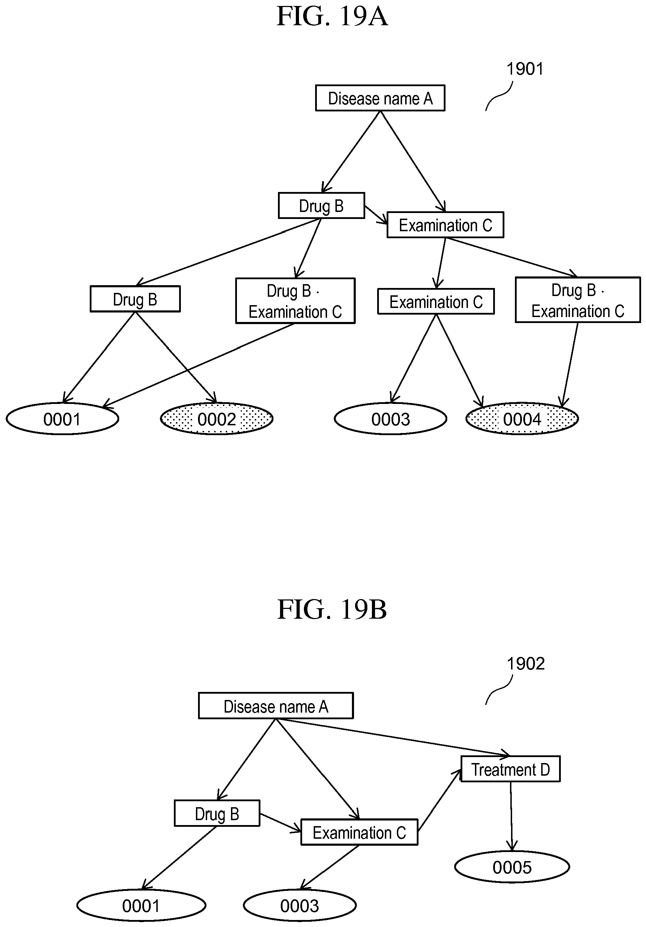

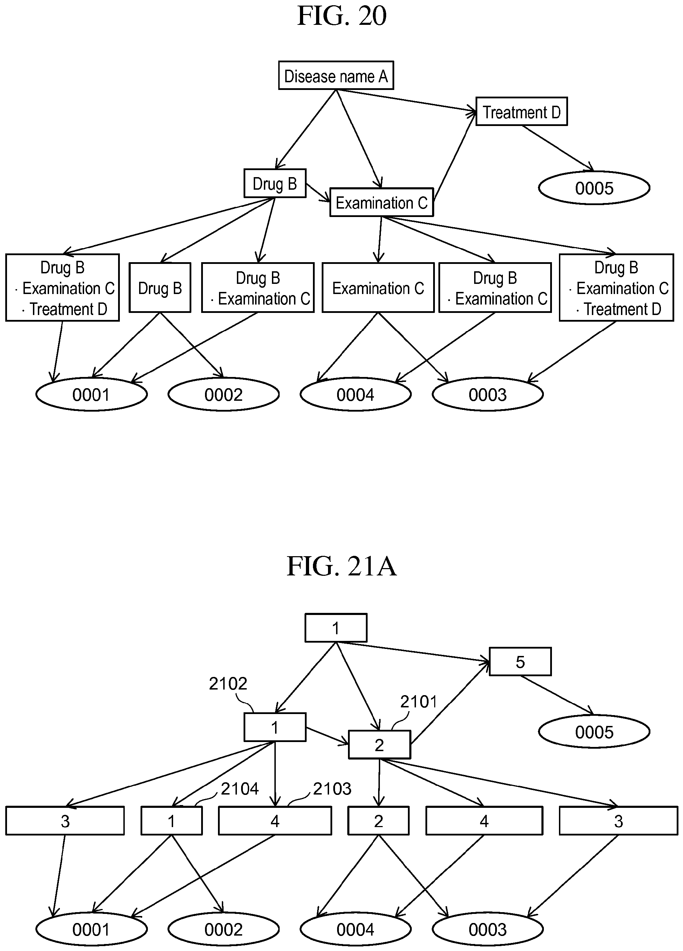

Fig. 19A and Fig. 19B are specific examples of the connected graph explained with reference to Fig. 17 and Fig. 18. Fig. 20 shows an example in which the two connected graphs shown in Fig. 19A and Fig. 19B are integrated.

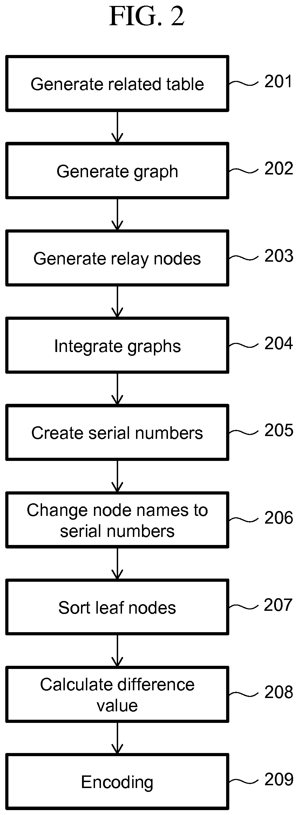

Fig. 2 shows a processing flow of the graph processing unit 104 executed by the CPU 2602 of the computer 2601. The graph processing unit 104 generates the graph (the reference tree) on the basis of the medical care data DB 105 and the medical care pattern DB 106 according to the processing flow of Fig. 2.

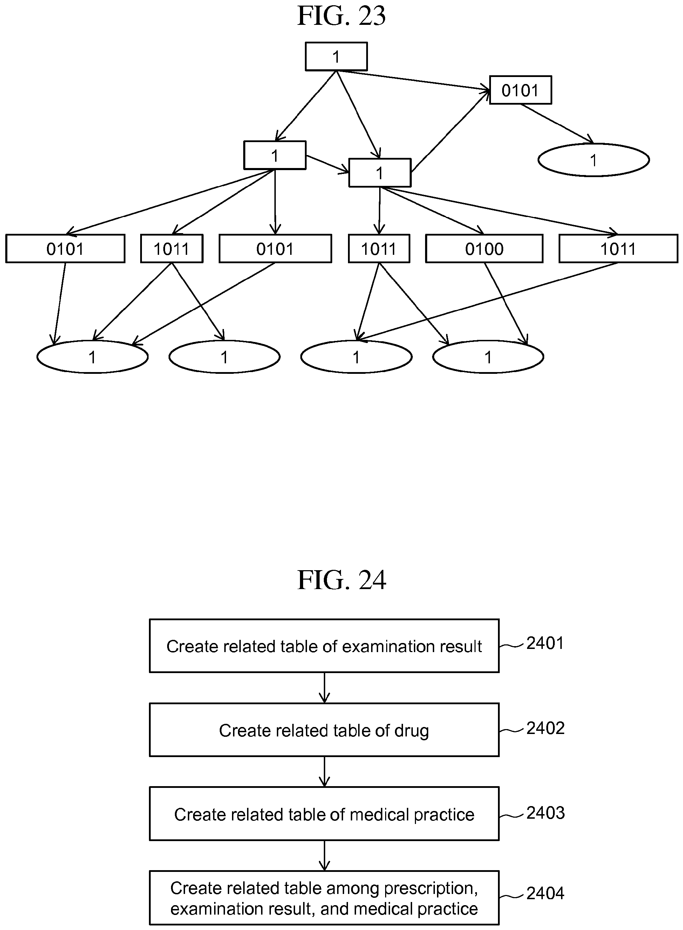

First, in step 201, the graph processing unit 104 creates the related table 900. This step is explained with reference to Fig. 24.

In step 2401, the graph processing unit 104 reads records one by one from the examination-adapted disease table 1100 and acquires the disease name 1101 and the examination name 1102. The graph processing unit 104 searches for the examination name 703 of the examination result table 700 on the basis of the acquired examination name 1102. If a matching record is present in the examination result table 700, the graph processing unit 104 acquires the order number 701, the patient ID 702, and the examination date 707 of the record.

Subsequently, the graph processing unit 104 searches for the patient ID 502 and the state name 503 in the episode table 500 on the basis of the acquired patient ID and the acquired disease name. If a matching record is present in the episode table 500, the graph processing unit 104 focuses on the start date 505 of the record. The graph processing unit 104 compares the acquired examination date and the start date.

For example, in some case, a plurality of disease names are diagnosed and a plurality of examinations are carried out on a certain patient. In this case, it is necessary to determine which disease names and which examinations are associated. If a certain disease name is diagnosed and an examination is carried out temporally after a result of the diagnosis, it is determined that the disease name and the examination may be associated. Note that other determination conditions may be used. A determination condition that an examination date is included in a range of a predetermined period based on the acquired start date may be used.

In this example, if the acquired examination date is temporally after the start date 505, the graph processing unit 104 acquires the episode number 501 and the target episode number 504 of the record. Thereafter, the graph processing unit 104 generates a new record in the related table 900. After incrementing the related number 901, the graph processing unit 104 records the acquired patient ID and the acquired episode number respectively in the patient ID 902 and the episode number 903. The graph processing unit 104 records the "target episode" concerning the table name 1 (904) and records the acquired target episode number" concerning the reference number 1 (905) of the table name 1 (904). The graph processing unit 104 records the "examination result" indicating the examination result table 700 concerning the table name 2 (906) and records the acquired order number concerning the reference number 2 (907).

In step 2402, the graph processing unit 104 reads records one by one from the drug-adapted disease table 1200 and acquires the disease name 1201 and the drug name 1202. The graph processing unit 104 searches for the drug name 803 of the prescription table 800 on the basis of the acquired drug name 1202. If a matching record is present in the prescription table 800, the graph processing unit 104 acquires the prescription number 801, the patient ID 802, and the prescription date 805 of the record.

Subsequently, the graph processing unit 104 searches for the patient ID 502 and the state name 503 in the episode table 500 on the basis of the acquired patient ID and the acquired disease name. If a matching record is present in the episode table 500, the graph processing unit 104 focuses on the start date 505 of the record. A reason for focusing on the start date is as explained above. If the acquired prescription date is temporally later than the start date 505, the graph processing unit 104 acquires the episode number 501 and the target episode number 504 of the record. Thereafter, the graph processing unit 104 generates a new record in the related table 900. After incrementing the related number 901, the graph processing unit 104 records the acquired patient ID and the acquired episode number respectively in the patient ID 902 and the episode number 903. The graph processing unit 104 records the "target episode" concerning the table name 1 (904) and records the acquired target episode number concerning the reference number 1 (905) of the table name 1 (904). The graph processing unit 104 records the "prescription" indicating the prescription table 800 concerning the table name 2 (906) and records the acquired prescription number concerning the reference number 2 (907).

In step 2403, the graph processing unit 104 generates a record of the related table 900 on the basis of the diagnostic treatment knowledge 1000 shown in Fig. 10. First, the graph processing unit 104 acquires a disease name with a section 1005 and acquires a drug name, a medical practice name, and the like from the medical care content sections 1003 and 1004. Processing performed when the drug name is acquired is the same as step 2402.

When the medical practice name is acquired, the graph processing unit 104 searches for the practice name 603 of the medical practice table 600 on the basis of the acquired medical practice name. If a matching record is present in the medical practice table 600, the graph processing unit 104 acquires the order number 601, the patient ID 602, and the start date 604 of the record.

The graph processing unit 104 searches for the patient ID 502 and the state name 503 in the episode table 500 on the basis of the acquired patient ID and the acquired disease name. If a matching record is present in the episode table 500, the graph processing unit 104 focuses on the start date 505 of the record. If the acquired start date 604 is temporally later than the start date 505, the graph processing unit 104 acquires the episode number 501 and the target episode number 504 of the record. Thereafter, the graph processing unit 104 generates a new record in the related table 900. After incrementing the related number 901, the graph processing unit 104 records the acquired patient ID and the acquired episode number respectively in the patient ID 902 and the episode number 903. The graph processing unit 104 records the "target episode" concerning the table name 1 (904) and records the acquired target episode number concerning the reference number 1 (905) of the table name 1 (904). The graph processing unit 104 records the "medical practice" indicating the medical practice table 600 concerning the table name 2 (906) and records the acquired order number concerning the reference number 2 (907).

In step 2404, the graph processing unit 104 checks a relation among the medical practice, the prescription, and the examination result on the basis of the diagnostic treatment knowledge 1000 shown in Fig. 10 and records the relation in the related table 900. In the example shown in Fig. 10, Action (1) of the medical care content section 1003 has to be carried out before Action (2) of the medical care content section 1004. That is, Fig. 10 indicates that, after drugs of aspirin and morphine are prescribed, intracardiac catheter, which is a medical practice, is carried out. The graph processing unit 104 checks such a relation between medical care contents on the basis of the diagnostic treatment knowledge 1000 and the related table 900.

First, the graph processing unit 104 focuses on the table name 2 (906) of a plurality of records having the same episode number 903 in the related table 900. When the table name 2 (906) is the "medical practice", the graph processing unit 104 collates the reference number 2 (907) of the table name 2 (906) with the order number 601 of the medical practice table 600 and acquires the patient ID 602 and the practice name 603 of a matching record. Similarly, When the table name 2 (906) is the "prescription", the graph processing unit 104 collates the reference number 2 (907) of the table name 2 (906) with the prescription number 801 of the prescription table 800 and acquires the patient ID 802 and the drug name 803 of a matching record. Similarly, when the table name 2 (906) is the "examination result", the graph processing unit 104 collates the reference number 2 (907) of the table name 2 (906) with the order number 701 of the examination result table 700 and acquires the patient ID 702 and the examination name 703 of a matching record.

The graph processing unit 104 determines whether the practice name 603, the drug name 803, and the examination name 703 acquired in the processing explained above are present in the Action (1) of the medical care content section 1003 and the Action (2) of the medical care content section 1004 shown in Fig. 10. If the practice name 603, the drug name 803, and the examination name 703 are present in the medical care content sections 1003 and 1004, the graph processing unit 104 adds a pair of the Actions in the related table 900 as a new record.

It is assumed that a pair of Actions in a certain diagnostic treatment knowledge 1000 is a drug name (corresponding to a prescription table) and an examination name (corresponding to an examination result table). It is assumed that the examination of the Action (2) has to be carried out after the prescription of the drug of the Action (1). After incrementing the related number 901, the graph processing unit 104 records the acquired patient ID and the acquired episode number respectively in the patient ID 902 and the episode number 903. The graph processing unit 104 records the "prescription" concerning the table name 1 (904) and records a prescription number concerning the reference number 1 (905) of the table name 1 (904). The graph processing unit 104 records the "examination result" concerning the table name 2 (906) and records an order number concerning the reference number 2 (907).

According to the processing flow shown in Fig. 24, it is possible to create, on the basis of the medial examination data DB 105 and the medical care pattern DB 106, information (the related table 900) for creating a graph (a reference tree) for efficiency of retrieval of medical care data. In particular, according to the processing flow shown in Fig. 24, it is possible to obtain information indicating a relation between episodes of patients and medical care contents and information indicating a relation among the medical care contents carried out on the patients.

Subsequently, in step 202, the graph processing unit 104 creates a graph on the basis of the related table 900 stored in the medical care data DB 105. First, the graph processing unit 104 captures records of the related table 900 in order, checks the episode number 903, groups a plurality of records having the same episode number 903. The graph processing unit 104 collects records, the table name (1) 904 of which is the "target episode", in the records included in the group of the same episode number. Concerning the records, the table name (1) 904 of which is the "target episode", the graph processing unit 104 acquires the reference number 1 (905) of the records. Subsequently, the graph processing unit 104 searches for a record, the target episode number 302 of which coincides with the acquired reference number 1 (905), in the target episode table 300. The graph processing unit 104 acquires the state name 303 of the record having the target episode number 302 coinciding with the reference number 1 (905). Subsequently, the graph processing unit 104 acquires a table name and a reference number of the table name from the table name (2) 906 and the reference number (2) 907. For example, if the table name (2) 906 is the "prescription", the graph processing unit 104 searches for a record having the proscription number 801 coinciding with the reference number (2) 907 in the prescription table 800 and acquires the drug name 803 concerning the reference number (2) 907. Note that the graph processing unit 104 performs the same processing when the table name (2) 906 is the "examination result" or the "medical practice".

The graph processing unit 104 creates a connected graph shown in Fig. 17 with the acquired state set as a root node. Subsequently, the graph processing unit 104 creates the intermediate notes 1704 and 1705 representing a prescription, a medical practice, or an examination result concerning the state name. The graph processing unit 104 creates, from the reference number (2) 907, the leaf nodes 1706 and 1707 corresponding to the intermediate nodes 1704 and 1705. Thereafter, the graph processing unit 104 connects the root node and the intermediate nodes 1704 and 1705 using pointers and connects the intermediate nodes 1704 and 1705 and the leaf nodes 1706 and 1707 using pointers.

In the related table 900, a record, in which a relation not directly related to a target episode is described, such as the "examination result" for checking a side effect concerning the "prescription" related to the drug name could be included. This is equivalent to a record in the case in which the table name (1) 904 and the table name (2) 906 in the related table 900 are a combination of two selected from the "medical practice", the "prescription", and the "examination result".

In this case, the graph processing unit 104 acquires, from a table corresponding to the table name (1) 904, a practice name, a drug name, or an examination name on the basis of the reference number 1 (905) and creates a node of the acquired practice name, drug name, or examination name. Similarly, the graph processing unit 104 creates nodes corresponding to the table name (2) 906 and the reference number 2 (907). To link the node concerning the table name (1) 904 to the node concerning the table name (2) 906, the former node retains a pointer of the latter node.

Subsequently, the graph processing unit 104 creates leaf nodes in which the reference number 1 (905) and the reference number (2) 907 are stored. Higher-order intermediate nodes retain pointers to the leaf nodes to thereby generate a link. That is, the intermediate node 1704 of the "intracardiac catheter" is linked to the leaf node 1706. The intermediate node 1705 of the "aspirin" is linked to the leaf node 1707.

Subsequently, in step 203, the graph processing unit 104 generates second intermediate nodes (relay nodes) in the graph created in step 202. Specifically, as shown in Fig. 18, when a plurality of intermediate nodes are associated by the link 1801 representing temporal anteriority and posteriority, the graph processing unit 104 creates, under the intermediate nodes, the second intermediate nodes (relay nodes) 1802 and 1804 representing a pattern of a combination of medical care contents of a plurality of intermediate nodes. Further, the graph processing unit 104 creates, under the second intermediate nodes 1802 and 1804, the leaf nodes 1803 and 1805 representing reference numbers for referring to the pattern of the combination of the medical care contents.

For example, it is assumed that the graph created in step 202 is in the state shown in Fig. 17. A link is present from the "intracardiac catheter", which is the practice name, to the "aspirin", which is the drug name. In this case, there are two cases concerning a medical practice of a node of a medical practice name; a case in which prescription of a drug of a node of the drug name is carried out after the medical practice and a case in which the prescription is not carried out. Therefore, the graph processing unit 104 creates two intermediate nodes anew. Specifically, the graph processing unit 104 creates the second intermediate node 1802 of the "intracardiac catheter/aspirin" indicating a case in which the "aspirin" is prescribed after the "intracardiac catheter" and the second intermediate node 1804 of the "intracardiac catheter" indicating a case in which only the "intracardiac catheter" is performed. The node 1800 of the "intracardiac catheter", which is a host node, retains pointers to the second intermediate nodes 1802 and 1804 to thereby generate a link. Subsequently, the graph processing unit 104 generates the leaf nodes 1803 and 1805 indicating the respective reference numbers of the second intermediate nodes 1802 and 1804. The second intermediate nodes 1802 and 1804 retain pointers of the leaf nodes 1803 and 1805 indicating the respective reference numbers. With such a graph structure in which the intermediate nodes are provided, it is possible to realize, as graph representation, the AND condition in the case in which the link is present between the nodes. Efficiency of retrieval is improved.

Subsequently, in step 204, the graph processing unit 104 searches for redundant portions in the graph group generated in step 203 and integrates the graph group. When there are a plurality of graphs concerning a certain symptom generated in step 203, the graph processing unit 104 integrates redundant portions of intermediate nodes among the plurality of graphs.

Processing of the integration is explained with reference to two graphs of a graph 1901 shown in Fig. 19A and a graph 1902 shown in Fig. 19B. In this example, the graph processing unit 104 searches for redundant portions between two graphs 1901 and 1902 in a range including a root node and intermediate nodes in a layer immediately under the root node and integrates the redundant portions. For example, in the graphs 1901 and 1902, nodes of a disease name A, a drug B, and an examination C and directions of links (arrows) among the nodes coincide with each other. The graph processing unit 104 integrates a plurality of graphs by superimposing the redundant portions of the intermediate nodes between the graphs in which root nodes coincide with each other. Fig. 20 is an example of an integrated graph. In step 204, the graph processing unit 104 performs this processing in all the graphs until the redundant portions of the graphs are eliminated. Note that, in this example, the redundant portions are searched in the range including the root node and the intermediate nodes in the layer immediately under the root node. However, the range for searching for the redundant portions is not limited to this and may be changed as appropriate.

Subsequently, in step 205, the graph processing unit 104 performs serial number generation for the node names in order to replace the node names of the nodes other than the leaf nodes with serial numbers in the graphs in which the processing in step 204 is performed. As a policy for giving serial numbers, when there are redundant practice names, drug names, examination names, or medical care patterns (combinations of medical practices, drugs, and examinations) in different target episodes, the serial numbers are allocated preferentially from the target episodes such that numbers of the target episodes are as close as possible. This is a device for reducing a difference values to be as small as possible in order to improve efficiency of compression in differential compression processing performed in the subsequent step 208 and step 209.

Processing performed when nodes of practice names, drug names, examination names, or medical care patterns (combinations of medical practices, drugs, and examinations) are redundant in graphs concerning different target episodes is explained. When there are a plurality of graphs concerning different target episodes (symptoms), the graph processing unit 104 gives numbers preferentially from graphs including more redundant intermediate nodes among the plurality of graphs.

For example, it is assumed that a graph including a state name A (a target episode A) as a root node includes a drug 1 and a medical practice 1, a graph including a state name B (a target episode B) as a root node includes the drug 1 and a medical practice 2, and a graph including a state name C (a target episode C) as a root node includes the drug 1, the medical practice 2, and a medical practice 3. In this case, two intermediate nodes are redundant between the target episode B and the target episode C. On the other hand, one intermediate node is redundant between the target episode A and the target episode B. Therefore, the serial number allocation in the target episodes B and C including a large number of redundant portions is prioritized. Therefore, the graph processing unit 104 allocates serial numbers "1", "2", and "3" respectively to the drug 1, the medical practice 2, and the medical practice 3.

Subsequently, the graph processing unit 104 allocates a serial number "4" to the medical practice 1 of the target episode A. In the target episode A, a difference between the drug 1 and the medical practice 1 is as large as 3. However, concerning the target episodes B and C, all differences are as small as 1. If the serial number allocation for the target episode A is performed after serial number allocation for other target episodes different from the target episodes B and C, the difference between the drug 1 and the medical practice 1 is a value larger than 3. Therefore, when there are redundant portions (practice names, drug names, examination names, or medical care patterns) in the graph, serial numbers are preferentially allocated from target episodes including the redundant portions. In the case of the example explained above, after the serial number allocation ends in the target episodes B and C, the serial number allocation to the target episode A is performed. Consequently, it is possible to allocate the serial numbers such that the numbers are as close as possible. It is possible to reduce difference values among the nodes. As a result, values of the difference values explained below are reduced as a whole. Short code words are allocated in the subsequent encoding. Therefore, it is possible to reduce a data amount of a graph (a reference trees) managed on the memory.

The serial numbers allocated by the procedure explained above are recorded in forms of serial number management tables shown in Fig. 13, Fig. 14, Fig. 15, and Fig. 16 according to each of practice names, drug names, examination names, and medical care patterns. The graph processing unit 104 records, in the forms of the serial number management tables shown in Fig. 13 to Fig. 16, relations between the allocated numbers and the medical care contents of the intermediate nodes.

Specifically, when a node is a medical care pattern, the graph processing unit 104 creates records in the medical care pattern serial number management table 1300. A state name serving as a root node of the graph is recorded in the root disease name 1301, a serial number is recorded in the number 1302, and a name of the medical care pattern is recorded in the medical care pattern 1303. When a node is an examination name, the graph processing unit 104 creates records in the examination serial number management table 1400. The examination name is recorded in the examination name 1401 and a serial number is recorded in the number 1402. When a node is a drug name, the graph processing unit 104 creates records in the drug serial number management table 1500. The drug name is recorded in the drug name 1501 and a serial number is recorded in the number 1502. Similarly, when a node is a practice name of a medical practice, the graph processing unit 104 creates records in the medical practice serial number management table 1600. The practice name is recorded in the practice name 1601 and a serial number is recorded in the number 1602.

Subsequently, in step 206, the graph processing unit 104 replaces names of the nodes other than the leaf nodes in the graph with the numbers of the serial number management tables shown in Fig. 13, Fig. 14, Fig. 15, and Fig. 16. Note that the graph processing unit 104 replaces the state name of the target episode with the serial number 301 of the target episode table 300. Fig. 21A shows an example in which the node names in Fig. 20 are replaced with the serial numbers.

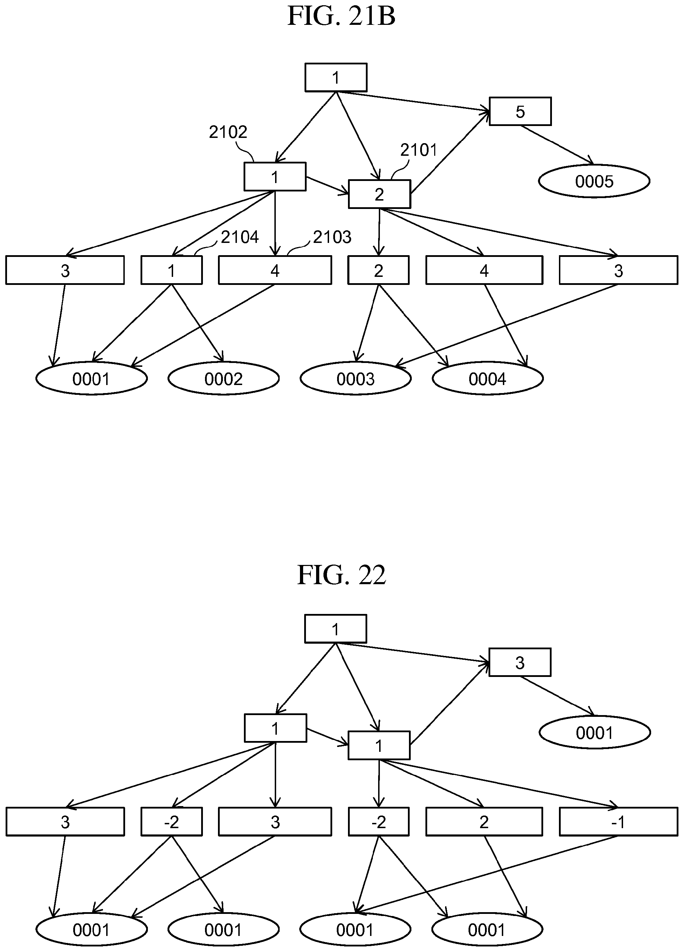

Subsequently, in step 207, concerning the graph after the processing in step 206 is performed, the graph processing unit 104 sorts the leaf nodes such that the reference numbers of the leaf nodes in the graph increase from the left to the right. This is for the purpose of, when a difference value is calculated, reducing the value of the difference to as small a value as possible. An effect of this is to reduce the data amount after the encoding as explained above. For example, Fig. 21B is an example in which the leaf nodes shown in Fig. 21A are sorted such that the reference numbers increase from the left to the right.

Subsequently, in step 208, the graph processing unit 104 calculates a difference between nodes adjacent to each other in the horizontal direction (i.e., nodes adjacent to each other in the same layer in the graph) in the graph after the processing in step 207 is performed and replaces the serial number or the reference number with a value of the difference. In the example shown in Fig. 21B, the graph processing unit 104 calculates a difference value in the horizontal direction to calculate a difference between the nodes 2101 and 2102 immediately under the root node. The graph processing unit 104 calculates a difference between the nodes 2103 and 2104 immediately under the nodes 2101 and 2102. Further, the graph processing unit 104 calculates a difference between the leaf nodes.

Specifically, concerning the nodes adjacent to each other in the horizontal direction, the graph processing unit 104 calculates a difference value by subtracting a serial number of the node on the left side from a serial number of the node on the right side. The graph processing unit 104 calculates a difference value "1" by subtracting a serial number "1" of the node 2102 from a serial number "2" of the node 2101. The difference value "1" is substituted in the node 2101 on the right side. Similarly, the graph processing unit 104 calculates difference values of the other nodes. Note that, since a difference cannot be calculated for the root node, the root node retains the original value. In each of the layers, a difference cannot be calculated for a node on the leftmost side. Therefore, the node retains the original value. Fig. 22 shows an example in which differences are calculated concerning the nodes shown in Fig. 21B.

Subsequently, in step 209, the graph processing unit 104 performs predetermined encoding on the difference values of the intermediate nodes calculated in step 208. Fig. 23 shows an example in which encoding is performed on the difference value of the nodes shown in Fig. 22. In this embodiment, the graph processing unit 104 performs delta encoding in step 209. A delta code is a variable length code and has a characteristic of allocating a short cord word to a small value and conversely allocating a long code word to a large value. Therefore, if the numbers after the subtraction are reduced in all the nodes, since short code words are allocated, it is possible to reduce a data amount as a whole. Note that the encoding processing is not limited to this. The data amount as a whole may be reduced by other encoding processing.

The graph 108 is created by the processing explained above. The graph processing unit 104 may store the graph 108 in the external storage device in advance. The graph 108 is read onto the memory during retrieval processing explained below. By using the graph created in this embodiment for the retrieval processing, efficiency of retrieval is improved even in medical care data having a complicated table structure.

In the past, an SQL optimizer often adopts hash combination in table combination including a large amount of data. If a hash table is stored on a memory, speed is high. However, if the memory is short, there is a problem in that speed is low to the contrary. On the other hand, with the graph 108 created in this embodiment, the data mount of the graph 108 is reduced as a whole. It is possible to manage the data even with a small memory amount compared with the past.

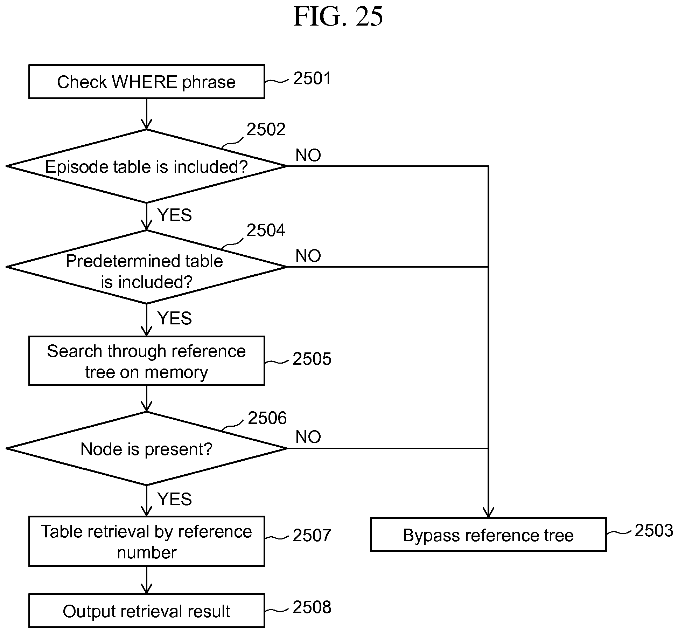

Fig. 25 is a flowchart for explaining the operation of the retrieval-expression analyzing unit 101, the retrieval-expression executing unit 102, and the retrieval output unit 103 during retrieval. In this retrieval processing, the retrieval is performed using the reference tree (the graph) created in the flowchart of Fig. 2. As shown in Fig. 1, the graph (the reference tree) 108 is read on the memory.

In step 2501, the retrieval-expression analyzing unit 101 receives an SQL sentence and performs an analysis of a WHERE phrase. Subsequently, in step 2502, the retrieval-expression analyzing unit 101 determines whether an expression concerning the episode table 500 is included in the WHERE phrase. When the expression concerning the episode table 500 is included, the retrieval-expression analyzing unit 101 proceeds to step 2504. Otherwise, the retrieval-expression analyzing unit 101 proceeds to step 2503.

Subsequently, in step 2504, the retrieval-expression analyzing unit 101 determines whether an expression concerning at least one of the medical practice table 600, the examination result table 700, and the prescription table 800 is included in the WHERE phrase. When the expression concerning the predetermined tables is included, the retrieval-expression analyzing unit 101 proceeds to step 2505. Otherwise, the retrieval-expression analyzing unit 101 proceeds to step 2503. In this way, the retrieval-expression analyzing unit 101 determines whether the table in the medical care data DB 105 is included in the retrieval conditions (WHERE phrase) and determines whether the graph 108 on the memory can be referred to. Consequently, the graph is prevented from being uselessly referred to.

Note that, when proceeding to step 2503, the retrieval-expression analyzing unit 101 determines to perform the retrieval processing without using the graph 108 on the memory and passes the SQL sentence to the retrieval-expression executing unit 102. The retrieval-expression executing unit 102 executes the passed SQL sentence and passes a retrieval result to the retrieval output unit 103.

In step 2505, the retrieval-expression analyzing unit 101 searches through the graph 108 on the memory from a connection relation of the episode table 500 and at least one table of the medical practice table 600, the examination result table 700, and the prescription table 800. Processing of the retrieval-expression analyzing unit 101 is explained below.

The processing is explained on the basis of a SQL sentence described below.

sql * from episode table, prescription table, examination result table where episode table. state name='myocardial infarct', medical practice table. practice name='intracardiac catheter';
First, concerning episode table. state name='myocardial infarct', the retrieval-expression analyzing unit 101 collates the state name 303 of the target episode table 300 and the "myocardial infarct" and acquires the serial number 301 corresponding to the "myocardial infarct". In the case of this example, the serial number 301 is "2". Subsequently, the retrieval-expression analyzing unit 101 finds a graph, a root node of which is "2", from a plurality of graphs 108 on the memory.

The processing is explained with reference to the medical practice table 600 as an example. However, the same applies to the examination result table 700 and the prescription table 800. Concerning medical practice table. practice name="intracardiac catheter", the retrieval-expression analyzing unit 101 collates the practice name 1601 of the medical practice serial number management table 1600 and the "intracardiac catheter" and acquires a serial number corresponding to the "intracardiac catheter". The retrieval-expression analyzing unit 101 checks whether the serial number appears in the nodes while traversing the graph (the graph, the root node of which is "2") and returning a delta-encoded node to the original serial number.

Subsequently, in step 2506, the retrieval-expression analyzing unit 101 determines presence of a node in step 2105. If a serial number of the "intracardiac catheter" is present in the node, the retrieval-expression analyzing unit 101 acquires a reference number concerning the medical practice from the leaf nodes and proceeds to step 2507. If the serial number is not present in the node, the retrieval-expression analyzing unit 101 proceeds to step 2503.

Subsequently, in step 2507, the retrieval-expression analyzing unit 101 searches for, on the basis of the reference number acquired in step 2506, records corresponding to the reference number in the target tables (the medical practice table 600, the examination result table 700, and the prescription table 800). Consequently, it is possible to search for positions of records matching the retrieval conditions (the WHERE phrase) of the SQL sentence.

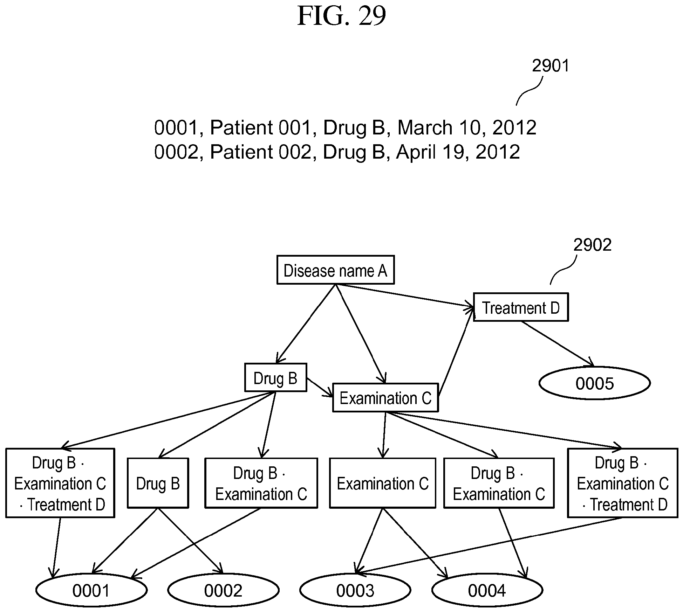

In step 2508, the retrieval-expression analyzing unit 101 passes a retrieval result in step 2507 to the retrieval output unit 103. Fig. 29 is an output example of the retrieval result. For example, the retrieval result is displayed on an output unit such as a display. A screen includes a retrieval-result output unit 2901 and a graph output unit 2902. The records matching the retrieval conditions are displayed on the retrieval-result output unit 2901. The graphs referred to by the retrieval-expression analyzing unit 101 are displayed on the graph output unit 2902. Display and hiding of the graphs in the graph output unit 2902 may be able to be switched by a not-shown option button or the like. The reference tree used for the retrieval is displayed in this way. This is useful because a ground of the retrieval processing is known.

### [Second Embodiment]

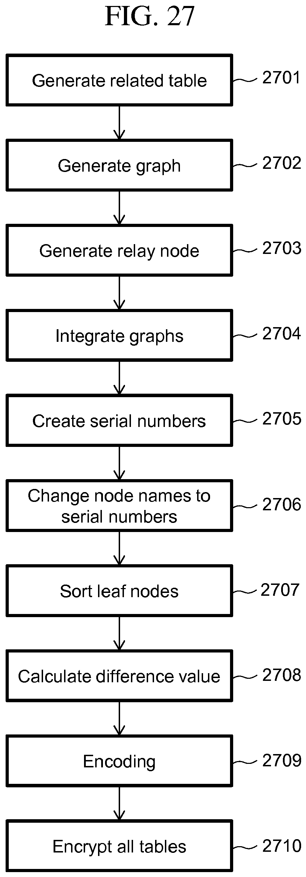

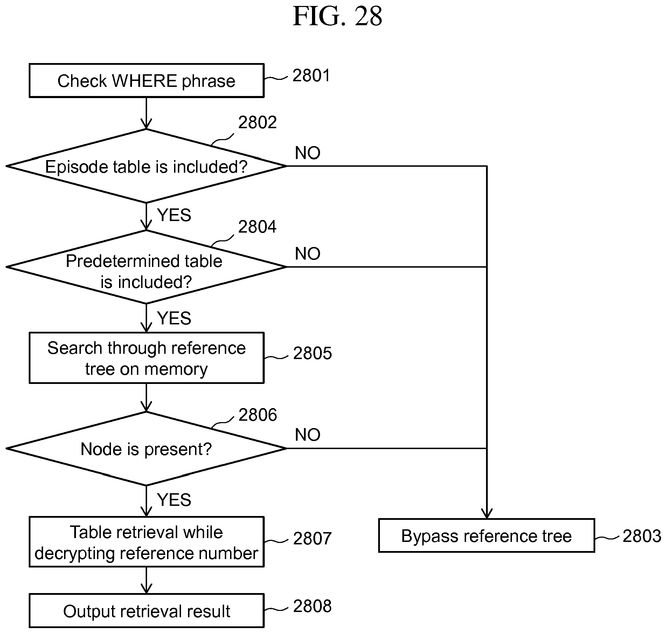

In this embodiment, processing for encrypting the data of the medical care data DB 105 is added to the processing in the first embodiment. This embodiment is explained with reference to Fig. 27 and Fig. 28.

In Fig. 27, step 2701 to step 2709 are the same as step 201 to step 209 in Fig. 2. In this embodiment, the graph processing unit 104 includes an encryption processing unit. After a graph is created in step 2701 to step 2709, in step 2710, the encryption processing unit of the graph processing unit 104 encrypts contents of the tables managed in the medical care data DB 105 shown in Fig. 3 to Fig. 9.

In this embodiment, a system of the encryption is not limited. However, all the records of the tables are encrypted by one common key. Consequently, security such as privacy protection for medical care data on a storage medium is improved.

Fig. 28 shows retrieval processing concerning the medical care data DB encrypted in the processing shown in Fig. 27. Steps 2801 to 2806 and 2808 are the same as steps 2501 to 2506 and 2508 in Fig. 25. In this embodiment, the retrieval-expression analyzing unit 101 includes a decryption processing unit. In step 2807, the retrieval-expression analyzing unit 101 searches for, on the basis of the reference number calculated in step 2806, records corresponding to the reference number in the target tables (the medical practice table 600, the examination result table 700, and the prescription table 800). The records of the medical care data DB 105 are encrypted in the processing shown in Fig. 27. Therefore, the decryption processing unit of the retrieval-expression analyzing unit 101 decrypts the reference number with the same common key. Consequently, the retrieval-expression analyzing unit 101 collates the records with the reference number calculated in step 2806 and acquires records corresponding to the reference number.

In step 2805 in Fig. 28, the records matching the retrieval conditions are searched using the graph (the reference tree). However, if this is performed on the encrypted medical care data DB 105, a load is large because it is necessary to decrypt all the encrypted data and perform combination processing among the tables. It is possible to greatly reduce the load of the decryption and the combination processing using the graph as in this embodiment. Therefore, it is possible to achieve both of enhancement of security such as privacy protection by the encryption and improvement of performance of the combination processing among the tables.

### Industrial Applicability

The invention is useful as a technique for improving efficiency of hospital management while improving the quality of diagnostic treatment by analyzing medical care data.

Note that the invention is not limited to the embodiments explained above. Various modifications are included in the invention. The embodiments are explained in detail in order to clearly explain the invention and are not always limited to embodiments including all the explained components. Further, a part of the components of a certain embodiment can be replaced with the components of another embodiment. The components of another embodiment can be added to the components of a certain embodiment. Other components can be added to, deleted from, and replaced with a part of the components of the embodiments.

A processor may realize, with software, the functions, the processing means, and the like of the medical care data retrieval system by interpreting and executing programs for realizing the functions, the processing means, and the like of the medical care data retrieval system. Information such as the programs, tables, and files for realizing the functions can be stored in storage devices such as a memory, a hard disk, and an SSD (Solid State Drive) or recording media such as an IC card, an SD card, and a DVD. The functions, the processing means, and the like of the medical care data retrieval system explained above may be realized with hardware by, for example, designing a part or all of the functions, the processing means, and the like as integrated circuits.

Control lines and information lines in the drawings considered to be necessary for explanation are shown. All control lines and information lines are not always shown in terms of a product. All the components may be connected to one another.

### Reference Signs List

- 101: retrieval-expression analyzing unit
- 102: retrieval-expression executing unit
- 103: retrieval output unit
- 104: graph processing unit
- 105: medical care data DB
- 106: medical care pattern DB
- 107: medical-care-pattern managing unit
- 108: graph (reference tree)
- 300: target episode table
- 400: patient table
- 500: episode table
- 600: medical practice table
- 700: examination result table
- 800: prescription table
- 900: related table
- 1000: diagnostic treatment knowledge
- 1100: examination-adapted disease table
- 1200: drug-adapted disease table
- 1300: medical care pattern serial number management table
- 1400: examination serial number management table
- 1500: drug serial number management table
- 1600: medical practice serial number management table
- 2601: computer
- 2602: CPU
- 2603: management terminal
- 2604: input/output interface
- 2605: memory
- 2606: communication interface
- 2607: external storage device

## Claims

1. A medical care data retrieval system comprising:
a storage device including a medical care data database in which medical care contents for symptoms of patients are stored and a medical care pattern database in which relations between the symptoms and the medical care contents are stored; and
a graph processing unit that creates, on the basis of the medical care data database and the medical care pattern database, a graph including a root node representing the symptom, at least one intermediate node representing a name of the medical care content, and at least one leaf node representing a reference number of the medical care content, wherein
when there are a plurality of the graphs concerning a certain symptom, the graph processing unit integrates redundant portions of the intermediate node among the plurality of graphs.

2. The medical care data retrieval system according to claim 1, wherein
the medical care pattern database includes a related table including information indicating the relations between the symptoms of the patients and the medical care contents and information indicating a relation among medical care contents carried out on the patients, and
the graph processing unit creates the graph using the related table.

3. The medical care data retrieval system according to claim 1, wherein, when a plurality of the intermediate nodes are associated by links, the graph processing unit creates, under the intermediate nodes, second intermediate nodes representing a pattern of a combination of the medical care contents of the plurality of intermediate nodes and creates, under the second intermediate nodes, leaf nodes representing reference numbers of the pattern of the combination.

4. The medical care data retrieval system according to claim 1, wherein the graph processing unit gives a number to the intermediate node on the graph and creates a serial number management table indicating a relation between the number and the medical care content of the intermediate node.

5. The medical care data retrieval system according to claim 4, wherein, when a plurality of the graphs concerning different symptoms are present, the graph processing unit gives the number preferentially from the graph including a large number of the intermediate nodes redundant among the plurality of graphs.

6. The medical care data retrieval system according to claim 4, wherein the graph processing unit sorts the leaf nodes such that the reference numbers are arranged in a predetermined order.

7. The medical care data retrieval system according to claim 4, wherein the graph processing unit calculates a difference value of the numbers between the intermediate nodes adjacent to each other in a same layer and substitutes the difference value in the intermediate nodes.

8. The medical care data retrieval system according to claim 7, wherein the graph processing unit encrypts the difference value.

9. The medical care data retrieval system according to claim 8, wherein the encoding is delta encoding.

10. The medical care data retrieval system according to claim 1, further comprising:
a memory that stores the graph; and
a retrieval-expression analyzing unit that searches for, referring to the graph on the memory, a position of a record in the medical care data database matching retrieval conditions of a retrieval sentence.

11. The medical care data retrieval system according to claim 10, wherein, when a table in the medical care data database is included in the retrieval conditions, the retrieval-expression analyzing unit refers to the graph on the memory.

12. The medical care data retrieval system according to claim 10, further comprising:
a retrieval output unit that outputs a retrieval result of the retrieval sentence; and
a display unit that displays the retrieval result and the graph.

13. The medical care data retrieval system according to claim 1, wherein the graph processing unit includes an encoding processing unit that encodes data in the medical care data database.

14. The medical care data retrieval system according to claim 13, further comprising:
a memory that stores the graph; and
a retrieval-expression analyzing unit that searches for, referring to the graph on the memory, a position of a record in the medical care data database matching retrieval conditions of a retrieval sentence.

15. The medical care data retrieval system according to claim 14, wherein the retrieval-expression analyzing unit includes a decryption processing unit that decrypts data in the medical care data database.
